Europäisches Patentamt

European Patent Office

Office européen des brevets

(19)

(11) Veröffentlichungsnummer: **0 270 852 B1**

# (12) EUROPÄISCHE PATENTSCHRIFT

(45) Veröffentlichungstag der Patentschrift: **08.01.92**

(21) Anmeldenummer: **87116477.8**

(22) Anmeldetag: **07.11.87**

Teilanmeldung 91105495.5 eingereicht am 07/11/87.

(51) Int. Cl.⁵: **C07C 43/04**, C07C 41/42, C07C 41/09

(54) **Verfahren zur Herstellung von reinem Dimethylether.**

(30) Priorität: **18.11.86 AU 65506/86**
**16.12.86 DE 3642845**

(43) Veröffentlichungstag der Anmeldung:
**15.06.88 Patentblatt 88/24**

(45) Bekanntmachung des Hinweises auf die
Patenterteilung:
**08.01.92 Patentblatt 92/02**

(84) Benannte Vertragsstaaten:
**AT BE CH DE ES FR GB GR IT LI LU NL SE**

(56) Entgegenhaltungen:
**EP-A- 0 099 676**
**EP-A- 0 124 078**

(73) Patentinhaber: **RWE-DEA Aktiengesellschaft**
**für Mineraloel und Chemie**
**Überseering 40**
**W-2000 Hamburg 60(DE)**

(72) Erfinder: **Dornhagen, Horst, Dr.**
**Auf dem Radacker 21**
**W-5047 Wesseling(DE)**
Erfinder: **Hammer, Hartmut, Dr.**
**Leyboldstrasse 21**
**W-5000 Köln(DE)**
Erfinder: **Meisenburg, Ewald**
**Höhenring 60**
**W-5357 Heimerzheim(DE)**
Erfinder: **Haas, Bernd, Dr.**
**Rabenweg 6**
**W-5047 Wesseling(DE)**

(74) Vertreter: **Schupfner, Gerhard D.**
**Müller, Schupfner & Gauger Karlstrasse 5**
**Postfach 17 53**
**W-2110 Buchholz/Nordheide(DE)**

## Beschreibung

Die Erfindung betrifft ein Verfahren zur Herstellung von reinem Dimethylether durch katalytische Dehydratisierung von Methanol und Reinigung des Dehydratisierungsprodukts durch Zuführung desselben in eine Destillationskolonne zur Gewinnung von reinem Dimethylether an bestimmten Böden dieser Kolonne und Entnahme des reinen Dimethylethers und von Verunreinigungen oberhalb bestimmter Böden der gleichen Kolonne.

Bis zur Entwicklung der Methanol-Niederdruck-Synthese-Verfahren wurde Dimethylether als Nebenprodukt der Methanol-Hochdruck-Synthese in einer Menge von etwa 2 - 5 Gew.% erhalten, bezogen auf die Gesamtmenge des Synthesereaktor-Ausgangsprodukts und bei der Gewinnung von Reinmethanol destillativ aus dem weitere Verunreinigungen enthaltenden Vorlauf abgetrennt.

Nach Einführung der Niederdruck-Methanolsynthese-Verfahren, bei denen keine nennenswerten Mengen an Dimethylether anfallen, wurden Syntheseverfahren zur Herstellung von Dimethylether durch katalytische Dehydratisierung von Methanol entwickelt.

Zahlreiche Verfahren sind in der Patentliteratur beschrieben worden. So lassen sich nach DE-PS 680 328 aliphatische Ether durch Erhitzen von Alkoholen in Gegenwart von Zinkchlorid erzeugen.

Weitere geeignete Katalysatoren zur Herstellung von Ethern aus Alkoholen sind gemäß GB-PS 332 756, GB-PS 350 010, GB-PS 403 402, US-PS 1 873 537 und FR-PS 701 335 Eisenchlorid, Kupfersulfat, Zinnchlorid, Manganchlorid, Aluminiumchlorid und -sulfat , Chromsulfat, Alaune, Thorium-Verbindungen, Aluminiumoxid, Titanoxid, Bariumoxid, Silicagel oder Aluminiumphosphat.

In "Industrial and Engineering Chemistry", Vol. 41, No. 12, S. 2928, 1949 wird die Verwendung von Bauxiten mit $SiO_2$ - Gehalten von 4,40 - 13,99 Gew.% beschrieben.

In US-PS 3 036 134 wird ein Aluminiumsilikat-Katalysator offenbart, zur Herstellung von Dimethylether aus Methanol, der ein $Al_2O_3$ : $SiO_2$ - Verhältnis von 1 Teil zu 1,35 bis 0,3 Teilen aufweist.

Auch die Synthese von Dimethylether direkt aus Synthesegas $(CO + H_2)$ ist beschrieben worden (DE-PS 23 62 944, DE-PS 27 57 788, und DE-PS 32 20 547).

Als technisch wichtigste Katalysatoren haben sich gemäß DE-PS 28 18 831, DE-OS 32 01 155, EP-A 0 099 676 und EP-A 0 124 078 insbesondere Aluminiumoxid- und Aluminiumsilikat-Katalysatoren mit und ohne Dotierung durchgesetzt.

In DE-PS 28 18 831 wird ein Katalysator zur Herstellung von Dimethylether offenbart, der jedes beliebige Aluminiumoxid-Grundmaterial mit genügend großer Oberfläche enthalten kann, in dem zusätzlich seltene Erden mit 1 - 30 Gew.% $SE_2O_3$ vorhanden sind.

Schließlich ist in EP-A 0 099 676 ein Katalysator beschrieben, der 1 - 20 Gew.% $SiO_2$, vorzugsweise 1 - 10 Gew.% $SiO_2$ und noch bevorzugter 6 Gew.% $SiO_2$ enthält.

Der hierbei anfallende rohe Dimethylether enthält das Reaktionswasser, nicht umgesetztes Methanol sowie kleine Mengen an Verunreinigungen, wie beispielsweise Methylformiat, Kohlenwasserstoffe, Amine und Sulfide, Carbonsäuren, Ester, Amide, Azetale u.a.

In diesen Synthese-Anlagen wird der rohe Dimethylether in zwei hintereinander geschalteten Destillationskolonnen aufgearbeitet, wobei in der ersten, unter Druck betriebenen Kolonne Dimethylether gewonnen wird und in der zweiten Kolonne nicht umgesetztes Methanol zurückgewonnen wird.

So wird in EP-A O 124 o78 ein Verfahren beschrieben, wonach in einer ersten unter Druck stehenden Kolonne, Dimethylether im Seitenabzug entnommen wird, während in einer zweiten unter niedrigerem Druck arbeitenden Kolonne über Kopf die zwischen Dimethylether und Methanol siedenden Verunreinigungen abgenommen werden. Methanol wird ebenfalls aus der 2. Kolonne im Seitenabzug entnommen.

Als Katalysator können $Al_2O_3$, $SiO_2$, Aluminiumsilikat und bevorzugt $\gamma$-Aluminiumoxid eingesetzt werden.

Obgleich bei diesem Verfahren ein gegenüber dem Stand der Technik reinerer Dimethylether gewonnen werden kann, hat es den erheblichen wirtschaftlichen Nachteil, daß nicht nur die erste, sondern auch die 2. Destillationskolonne mit hoher Bodenzahl ausgestattet sein muß, so daß sowohl hohe Investitionskosten als auch insbesondere hohe Betriebskosten entstehen und daß ferner die Gefahr besteht, daß die zwischen Dimethylether und Methanol siedenden Verunreinigungen nicht vollständig in die 2. Kolonne gelangen, sondern nach Anreicherung in der 1. Kolonne mit dem Dimethylether abgezogen werden, so daß keine Geruchsfreiheit vorliegt.

Da Dimethylether als Treibmittel für Aerosolsprays zunehmend an Bedeutung gewinnt, werden für bestimmte Anwendungen sehr hohe Reinheitsanforderungen an dieses Produkt gestellt. So dürfen insbesondere für die kosmetische, human- und Haushalts-Anwendung keine irritierenden Substanzen im Dimethylether enthalten sein. Ferner muß der Dimethylether hierbei frei bzw. weitgehend frei von Geruchsstoffen sein.

Es bestand daher die Aufgabe, einen hochreinen Dimethylether zu erzeugen nach einem gegenüber dem Stand der Technik wirtschaftlicheren Verfahren und andererseits den Dimethylether möglichst quantitativ in hochreiner Form zu erzeugen, so daß er für die genannten Anwendungen geeignet ist.

Die Lösung dieser Aufgabe ist der Anmelderin in hervorragender Weise duch das erfindungsgemäße Verfahren gelungen durch die kontinuierliche Herstellung von reinem Dimethylether durch katalytische Dehydratisierung von Methanol bei einer Temperatur von 140 - 500 $^\circ$C und einem Druck von 1 bis 50 bar und destillative Aufarbeitung des Dehydratisierungsprodukts (Ausgangsprodukt aus dem Synthesereaktor) dadurch gekennzeichnet, daß das Dehydratisierungsprodukt in die Kolonne zur Gewinnung von reinem Dimethylether unterhalb des 25. Bodens (vom Kopf der Kolonne) an einem oder mehreren Böden in die Kolonne eingesetzt wird, daß der reine Dimethylether ab dem des 15. Boden, bevorzugt oberhalb des 10. Bodens (vom Kopf der Kolonne) an einem Boden oder mehreren Böden abgezogen wird, daß ein Verunreinigungen, die höher als Dimethylether und niedriger als Methanol sieden, enthaltendes Destillat an einem Boden oder mehreren Böden abgezogen wird, der die um mindestens 5 Böden oberhalb des (obersten) Zulaufs des den Dimethylether enthaltenden Einsatzgemisches und um mindestens einen Boden unterhalb des 15. Bodens (vom Kopf der Kolonne) liegt (liegen), und daß das Dehydratisierungsprodukt mindestens 1 Boden oberhalb des Kolonnensumpfes zugeführt wird, wobei bei einem Einsatzprodukt, das 1-5 Gew.% Dimethylether, ein Rückflußverhältnis von 1:1 bis 1:25 gefahren wird, bei einem Einsatzprodukt, das 2o-8o Gew.% Dimethylether enthält, ein Rückflußverhältnis von 1:0,4 bis 1:5 gefahren wird, bevorzugt von 1:1 bis 1:2,5, bei einem Einsatzprodukt, das 6-19 Gew.% Dimethylether enthält, ein Rückflußverhältnis von 1:0,4 bis 1:25 und bei einem Einsatzprodukt, das >8o Gew.% und <99 Gew.% Dimethylether und die gleichen Gemischkomponenten enthält ein Rückflußverhältnis von 1:0,01 bis 1:5 gefahren wird.

Die Bestimmung geruchsbelästigender Substanzen erfolgt noch immer überwiegend empirisch, wobei direkte sensorische Messungen durch ein geübtes Team erfolgen. So wurde beispielsweise die Belästigungsschwelle "BS" für $H_2S$ durch ein Testkollektiv von 15o Personen auf 45 $\mu g/m^3$ ermittelt. (Schriftenreihe der Landesanstalt für Immissionsschutz des Landes Nordrhein-Westfalen, Heft 49 (1979) S.77).

In den Fällen in denen die Geruchsschwelle im durch Instrumente messbaren Bereich liegt, können Geruchsschwellen bzw. Belästigungsschwellen auch durch Messmethoden wie Gaschromatographie, elektrische Leitfähigkeit, Photometrie oder Fluoreszenzmessung bestimmt werden ("Erdöl und Kohle-Erdgas-Petrochemie", Bd. 32, Heft 2, Febr. 1975, S. 86).

Die Geruchsbestimmungen im Falle der vorliegenden Erfindung beruhen auf der sensorischen Methode.

Sowohl Rohmethanol aus einer Methanolsyntheseanlage als auch katalytisch aus Methanol hergestellter Dimethylether enthalten wie bereits oben ausgeführt, zahlreiche Verunreinigungen, die teilweise sehr geruchsintensiv sind. Während in Methanol-Hochdrucksyntheseanlagen ein Rohmethanol anfällt, in dem im allgemeinen 2-3, jedoch bis zu 5 Gew.% Dimethylether enthalten sein können, werden bei Dimethylethersynthesen aus Roh- oder Reinmethanol je nach Fahrweise im allgemeinen 2o bis 8o Gew.% Dimethylether aus dem eingesetzten Methanol am Reaktorausgang erhalten. Zusätzlich sind im rohen Dimethylether die oben genannten Verunreinigungen, Reaktionswasser und nicht umgesetztes Methanol enthalten.

Da die Siedepunkte der Verunreinigungen, wie beispielsweise von Dimethylamin (Kp 6,9 $^\circ$C), Dimethylsulfid (Kp. 37,3 $^\circ$C), Methylmercaptan (Kp.5,8 $^\circ$C), Ameisensäure (Kp.1oo,75 $^\circ$C), Ameisensäuremethylester (Kp.31,5 $^\circ$C), Formaldehyd (Kp.-21 $^\circ$C), Formaldehyddimethylacetal (Kp.45,5 $^\circ$C) oder Essigsäuremethylester (Kp.56,95 $^\circ$C) sowie ihre Löslichkeiten und Dampfdrucke im Produktgemisch sehr unterschiedlich sind und die subjektiv wahrgenommene Geruchsintensität der einzelnen Verunreinigungen ebenfalls sehr unterschiedlich ist und zusätzlich eine Reihe azeotroper Gemische gebildet werden, wird die Lösung der Aufgabe, hochreinen Dimethylether in hohen Ausbeuten nach einem gegenüber dem Stand der Technik wirtschaftlicheren Verfahren zu erzeugen, sehr erschwert.

Die Anmelderin hat daher in mehrjährigen Untersuchungen durch zahlreiche Testreihen in Labor, Technikum und technischer Anlage überraschend gefunden, daß hochreiner Dimethylether praktisch quantitativ in einer besonders wirtschaftlichen Weise durch die erfindungsgemäße destillative Reinigung erhalten werden kann.

In Fig. 1 ist beispielhaft eine Anlage zur Erzeugung und Reinigung von Dimethylether dargestellt.

In Fig. 2 ist beispielhaft eine Destillationskolonne für die Gewinnung von reinem Dimethylether dargestellt.

In Fig.3 ist beispielhaft diese Kolonne mit einem Vorlauf-Seitenstripper dargestellt.

Die in den Ansprüchen 2,3 und 4 offenbarten Katalysatoren sind Katalysatoren vom $\gamma$-$Al_2O_3$-Typ. Sie enthalten im Unterschied zu den Katalysatoren des Standes der Technik nur sehr geringe Mengen $SiO_2$ und führen zu wesentlich besseren Ergebnissen als die bekannten Katalysatoren. Der $SiO_2$-Gehalt ist o,oo1 bis 3 Gew.%. Bevorzugt ist eine Menge an $SiO_2$ von o,oo1 bis o,5 Gew.% und besonders bevorzugt von o,oo1 bis o,2 Gew.%

SiO$_2$.

Die $\gamma$-Al$_2$O$_3$-Katalysatoren können zusätzlich weitere Komponenten in kleinen und Kleinst-Mengen enthalten, wie z.B. Na$_2$O oder andere Alkali- und Erdalkalioxide, Alkali-, Erdalkali- oder Aluminiumsulfate, Eisenoxid, Kobaltoxid, Nickeloxid und andere Verbindungen.

Die Umsetzung an diesen Katalysatoren erfolgt bei einer Temperatur von l4o bis 5oo°C, bevorzugt von l5o bis 45o°C und einem Druck von l bis 5o bar, bevorzugt von l bis 25 bar. Die Umsetzung kann in der Gas- oder Flüssigphase erfolgen, bevorzugt ist die Umsetzung in der Gasphase. Es ist von Vorteil, die Drücke im Synthesereaktor und der destillativen Aufarbeitung des Dimethylethers aneinander anzupassen. Es wird bei Katalysatorbelastungen von o,2 - l6 l/l h, bevorzugt von o,5 - l3,5 l/l h gearbeitet.

Obgleich mit dem erfindungsgemäßen Verfahren diskontinuierlich gearbeitet werden kann, ist die kontinuierliche Fahrweise bevorzugt.

Als Reaktoren für die erfindungsgemäße Umsetzung können beispielsweise die dem Stand der Technik entsprechenden Festbett-, Fließbett- und Wirbelbett-Reaktoren eingesetzt werden. Auch abgewandelte Reaktoren, die für Umsetzungen in Gegenwart von Katalysatoren geeignet sind, können erfindungsgemäß eingesetzt werden.

Die Umsetzung in Gegenwart der $\gamma$-Al$_2$O$_3$-Katalysatoren kann im geraden Durchgang kinetisch oder thermodynamisch bestimmt sein in Abhängigkeit von den Reaktionsparametern, hierbei können jeweils die entsprechenden Mengen Dimethylether neben nicht umgesetztem Methanol am Reaktorausgang erhalten werden.

Die Untersuchungen der Anmelderin haben zu dem Ergebnis geführt, daß zur Lösung der Aufgabe, auf besonders wirtschaftliche Weise hochreinen geruchsfreien Dimethylether mit nahezu quantitativer Ausbeute gewinnen zu können, die Zuführung des Einsatzproduktes in die Destillationskolonne zur Gewinnung reinen Dimethylethers unterhalb des 25. Bodens (vom Kopf der Kolonne) an einem oder mehreren Böden erfolgen muß. Wird das Einsatzprodukt an einem höheren Boden zugeführt, so erhält man nicht die gewünschte Reinheit, insbesondere nicht die gewünschte Geruchsfreiheit. Das Einsatzprodukt kann erfindungsgemäß auch an mehreren Böden je nach Bodenzahl der Kolonne zwischen dem 25. und 5o. Boden (vom Kopf der Kolonne) zugeführt werden. So kann beispielsweise bei einer Kolonne, die insgesamt 6o Böden enthält, zwischen dem 32. und 46. Boden das Einsatzprodukt zugeführt werden.

Da die Zuführung mindestens l Boden, bevorzugt 5 Böden und besonders bevorzugt lo Böden oberhalb des Kolonnensumpfes liegen muß, kann (können) die Zuführung(en) im Bodenbereich unterhalb des 25. Bodens (vom Kopf der Kolonne) und oberhalb des l., bevorzugt 5. und besonders bevorzugt lo. Bodens (vom Sumpf der Kolonne) gewählt werden. Ferner muß erfindungsgemäß der reine Dimethylether oberhalb des l5. Bodens, bevorzugt oberhalb des lo. Bodens (vom Kopf der Kolonne) an einem oder mehreren Böden abgezogen werden. Dies kann beispielsweise der 6. Boden sein, es kann jedoch auch der l. bis 5. Boden bzw. ein Boden zwischen dem 6. und l5. Boden sein, es kann jedoch ggfs. auch das Kopfkondensat abgezogen werden. Es können auch mehrere Böden in diesen Bereichen sein.

Die Untersuchungen der Anmelderin haben ferner ergeben, daß Komponenten mit Siedepunkten zwischen denen von Methanol und Dimethylether als Destillat in der gleichen Kolonne an einem Boden oder mehreren Böden abgezogen werden können, der bzw. die um mindestens 5 Böden oberhalb des (obersten) Zulaufs für das den verunreinigten Dimethylether enthaltenden Einsatzprodukts und/oder des Einsatzgemischs das Dimethylether, Methanol und Verunreinigungen enthält, jedoch unterhalb des l5. Bodens (vom Kopf der Kolonne) liegt bzw. liegen.

Setzt man ein Einsatzprodukt ein, das beispielsweise 1 - 5 Gew.% Dimethylether enthält, neben Methanol, sonstigen Komponenten mit Siedepunkten zwischen denen von Methanol und Dimethylether und ggfs. Wasser und sonstigen Sauerstoff enthaltenden Kohlenwasserstoffen wie beispielsweise Alkoholen mit einer Anzahl C-Atomen > 1, so wird die Kolonne mit einem Rückflußverhältnis von 1 : 1 bis 1 : 25 betrieben in Abhängigkeit von dem Dimethyletheranteil. So kann beispielsweise bei einem Dimethylethergehalt von 1 Gew.% das Rückflußverhältnis 1 : 20 betragen. Ein Rückflußverhältnis von beispielsweise 1 : 1 bedeutet, daß die Menge an abgezogenem Dimethylether = 1 Teil ist und die Dampfmenge zur Kondensation am Kopf der Kolonne = 1 + 1 Teile ist, (1. Zahl stellt die abgezogen Menge dar). Ein Rückflußverhältnis von 1 : 5-8 ist beispielsweise bei zugt bei einem Dimethylethergehalt von 3 -4 Gew.%. Diese Dimethylethermenge entspricht den Gehalten, wie sie bei Hochdruck-Methanolsynthesenanfallen. Der Dimethylether kann jedoch nur dann in hochreiner, nahezu quantitativer Menge gewonnen werden, wenn man die erfindungsgemäß genannte Zuführung und die erfindungsgemäßen Abnahmestellen von Dimethylether und Verunreinigungen enthaltendem Destillat wählt. Enthält das Einsatzgemisch beispielsweise 20 - 80 Gew.% Dimethylether sowie Methanol und sonstige Komponenten mit Siedepunkten zwischen denen von Methanol und Dimethylether und ggfs. Wasser und sonstige Sauerstoff enthaltende Kohlenwasserstoffe, wie beispielsweise Alkohole mit einer Anzahl C-Atomen >1, so ist das Rückflußverhältnis auf 1 : 0,4 bis 1 : 5, bevorzugt von 1 : 1 bis 1 : 2,5 einzustellen je nach Dimethylethergehalt. Beispielsweise stellt man bei einem Gemisch, das 60 Gew.% Dimethylether und 15 Gew.% Methanol enthält, neben Wasser und Verunreinigungen, ein Rückflußverhältnis von 1 : 1,5 bis 1 : 2,5 ein.

Solche Einsatzgemische sind typische Gemische, wie sie bei der katalytischen Gewinnung von Dimethylether aus Methanol mit $Al_2O_3$- bzw. $Al_2O_3/SiO_2$-Katalysatoren im geraden Durchgang am Ausgang des Synthesereaktors anfallen.

Bei Dimethylethergehalten, die zwischen 5 und 20 Gew.% liegen bzw. oberhalb 80 Gew.% liegen, sind die erfindungsgemäßen Rückflußverhältnisse auf der Basis der angegebenen Werte zu wählen wie z. B. für Gehalte von 5 - 20 Gew.% zwischen einem Rückflußverhältnis von 1 : 0,4 bis 1 : 25 zu wählen, bzw. bei >80 Gew.% auch bei einem Verhältnis 1 : 0,01 bis 1 : 5.

Die Destillationskolonne zur Gewinnung des reinen Dimethylethers wird im allgemeinen bei einem Druck von 5 - 10 bar betrieben, wobei im Falle eines vorgeschalteten Synthesereaktors bevorzugt eine Anpassung an den Druck im Reaktor erfolgt. Drucke außerhalb dieses Bereichs sind erfindungsgemäß ebenfalls möglich.

Der Durchsatz wird wie üblich, durch die Auslegung der Kolonne, die Wärmezufuhr und das Rückflußverhältnis bestimmt.

Zur nahezu quantitativen Gewinnung des reinen Dimethylethers, insbesondere bei kleinen Gehalten des Dimethylethers im Einsatzprodukt kann erfindungsgemäß das über Kopf der Kolonne gehende Abgasgemisch, das im allgemeinen $CO_2$, $N_2$, Kohlenwasserstoffe und noch kleine Anteile an Dimethylether enthält, gewaschen werden. Die den Dimethylether enthaltende Waschflüssigkeit kann in die Kolonne oder in den Dimethylether-Synthesereaktor rückgeführt werden.

Geeignete Waschflüssigkeiten sind beispielsweise Methanol oder Sumpfprodukte der Dimethylether-Destillationskolonne. Das Waschen kann im Gleich- oder Gegenstrom erfolgen, bevorzugt jedoch im Gegenstrom.

Ferner kann das die Verunreinigungen enthaltende Destillat zusätzlich in einem Seitenstripper gestripped werden, wobei das Stripp-Produkt wieder in die Kolonne zurückgeführt wird. Hierdurch kann ggfs. an dieser Stelle ausgetretener Dimethylether nahezu vollständig rückgeführt werden.

· Wie für den Fachmann deutlich ist, wird erfindungsgemäß, das bei 64,7° C siedende Methanol von Wasser in einer 2. Kolonne, die als Abtreibkolonne betrieben wird, abgetrennt.

Nach dem erfindungsgemäßen Verfahren können Destillationskolonnen nach dem Stand der Technik entsprechend der Anlagenkapazität eingesetzt werden.

Die Böden können die dem Stand der Technik entsprechenden Böden sein, wie beispielsweise Ventilböden, Siebböden, Glockenböden u.a. Grundsätzlich können auch Füllkörper oder Packungen wie beispielsweise keramische Materialien, Glasmaterialien, Packungen aus Draht und dergleichen als Füllung für die Destillationskolonne verwendet werden, wobei die erfindungsgemäße Einsatzproduktzuführung, bzw. die Entnahmestellen für Dimethylether und Vorlauf gemäß den erforderlichen Bodenzahlen errechnet werden können.

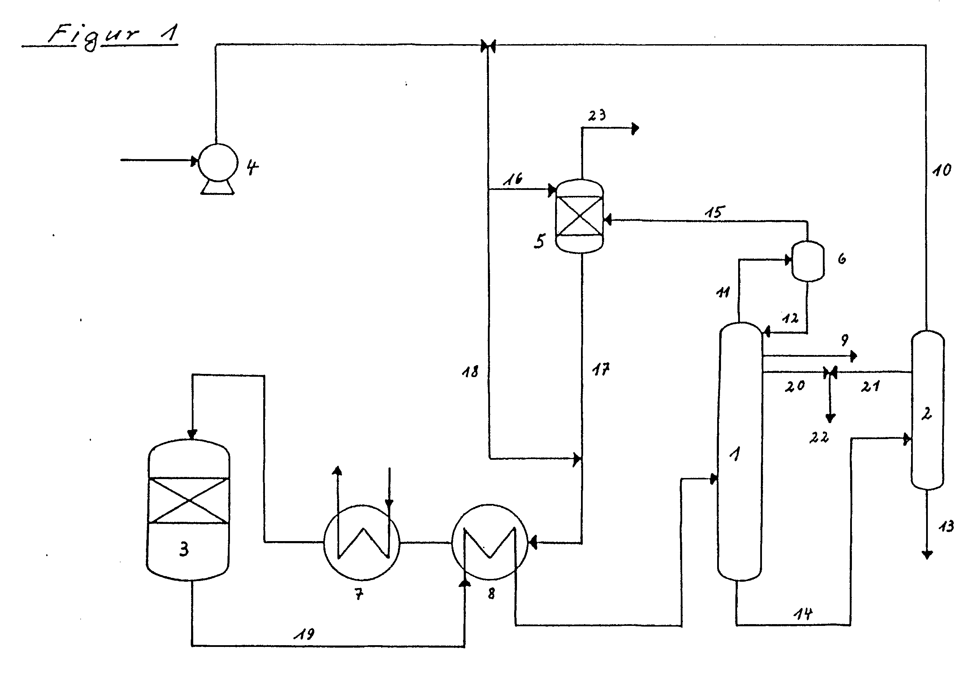

Die Erfindung wird mit Hilfe der Figuren 1 bis 3 näher erläutert.

In Figur 1 stellt (3) den Dehydratisierungsraktor dar. Über Pumpe (4), Leitung (18) und Wärmetauscher (8) und (7) wird frisches Methanol dem Reaktor zugeführt. (1) stellt die Destillationskolonne zur Gewinnung des reinen Dimethylethers dar. Die Kolonne wird im allgemeinen in einem Druckbereich betrieben, der dem Druck in Synthesereaktor (3) angepasst ist, üblicherweise bei etwas niedrigerem Druck. Liegt beispielsweise der Synthesedruck bei 8 - 12 bar, so kann die Kolonne beispielsweise bei 6 - 11,5 bar betrieben werden. Diese Zahlen sind jedoch nicht als einschränkend anzusehen. Im allgemeinen wird jedoch bevorzugt bei einer Druckdifferenz von 0 bis zu 10 bar gearbeitet. Der Dimethylether wird bei (9) in hochreiner Form abgezogen. Kopfgas gelangt über (11) in Kondensator (6). Rückfluß fließt über (12) wieder auf Kolonne (1). Das Abgas fließt über (15) in Wäscher(5), in dem es mit Methanol (16) gewaschen wird. Grundsätzlich können auch andere Waschflüssigkeiten eingesetzt werden, wie beispielsweise Rohmethanol oder Sumpf der Kolonne 1, wobei im letzteren Fall die den Dimethylether enthaltende Waschflüssigkeit wieder der Kolonne 1 zugeführt werden kann. Kleine Mengen Dimethylether gelangen über (17) zusammen mit Waschmethanol in den Synthesereaktor (3). Das Abgas entweicht bei (23). Das Syntheseprodukt (19) fließt über (8) nach (1). Bei (20) werden die zwischen Methanol und Dimethylether siedenden Verunreinigungen abgezogen und gelangen über (22) beispielsweise zur Verbrennung. Über (14) fließt der im wesentlichen Methanol und Wasser enthaltende Sumpf in Abtreibkolonne (2). Diese wird im allgemeinen bei Normaldruck betrieben, kann jedoch prinzipiell auch bei etwas erhöhtem oder etwas niedrigerem Druck betrieben werden, jedoch im allgemeinen unterhalb des Druckes der Kolonne (1). Über (10) wird Methanol abgezogen und zum Synthesereaktor zurückgeführt. Aus dem Sumpf wird über (13) Abwasser abgezogen. Über (21) können im Bedarfsfall Verunreinigungen entnommen werden mit höheren Siedepunkten als dem des Methanols.

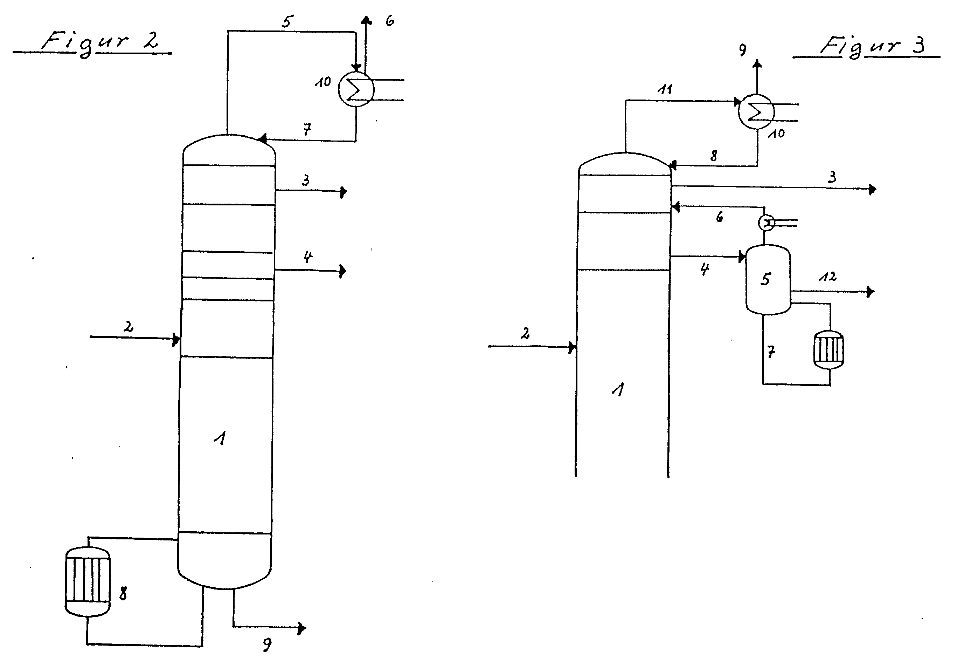

In Figur 2 stellt (1) die Destillationskolonne zur Gewinnung von reinem Dimethylether dar. Das Einsatzprodukt wird über (2) zugeführt. Der reine Dimethylether wird bei (3) entnommen. Die Verunreinigungen werden bei (4) abgezogen. Das Kopfprodukt fließt über (5) zum Kondensator (10). Rückfluß fließt über (7) zu Kolonne (1). Abgas entweicht bei (6). (8) stellt den Reboiler-Kreislauf dar. Bei (9) wird der Sumpf abgezogen.

In Figur 3 fließt das die Verunreinigungen enthaltende Destillat über (4) zum Stripper (5), der mit Reboiler-Kreislauf (7) ausgerüstet ist.

Abgestrippter Dimethylether fließt über (6) zur Kolonne (1) zurück.
Bei (2) wird Einsatzprodukt zugeführt. Bei (3) wird reiner Dimethylether entnommen. Kopfgas gelangt über (11) in Kondensator (10). Rückfluß fließt über (8) zur Kolonne (1). Abgas entweicht bei (9). Bei (12) werden die von Dimethylether befreiten Verunreinigungen abgenommen.

Gemäß vorliegender Erfindung wird aus der Destillationskolonne der hochreine Dimethylether praktisch quantitativ gewonnen. Der Dimethylether ist geruchsfrei, enthält weniger als 10 ppm Methanol, maximal 0,1 Gewichtsprozent Kohlenwasserstoffe und besitzt eine Reinheit von bis zu 99,9 Gew.% Dimethylether. Der erfindungsgemäß gewonnene Dimethylether ist für jede Anwendung im Aerosolbereich hervorragend geeignet.

Ferner wird gegenüber dem Stand der Technik nur eine Kolonne mit hoher Trennleistung benötigt, während die 2. Kolonne nur eine geringe Trennleistung benötigt, um Methanol von Wasser zu trennen. Zusätzlich erlaubt das erfindungsgemäße Verfahren, in der 2. Kolonne auch ggfs. zwischen Methanol und Wasser siedende Verunreinigungen abzutrennen, so daß ein an Verunreinigungen ärmeres, leichter aufarbeitbares Abwasser anfällt. Obgleich der Betrieb zusätzlicher Kolonnen grundsätzlich möglich ist, würde eine solche Fahrweise die Wirtschaftlichkeit des Verfahrens beeinträchtigen.

Beispiele

Die Aufarbeitung des Reaktorausgangsproduktes (3000 kg/h Einsatzprodukt mit 65 Gew.-% Dimethylether) in den folgenden Beispielen 1 bis 9 erfolgte in 2 hintereinander geschalteten kontinuierlich arbeitenden Destillationskolonnen, von denen die erste mit 50 Ventilböden und die 2. mit Raschigringen ausgerüstet war. Der reine Dimethylether wurde in der ersten Kolonne bei 7 bar am 12. Boden (vom Kopf der Kolonne) bei einem Rückflußverhältnis von 1:2 gewonnen. Das Einsatzprodukt wurde am 27. Boden (vom Kopf der Kolonne) zugeführt. Die Verunreinigungen wurden am 22. Boden abgezogen. In der 2. Kolonne wurde nicht umgesetztes Methanol zurückgewonnen. Die Synthese wurde bei einer Temperatur von 270-290 °C und einem Druck von 10 bar durchgeführt.

In Beispiel 1 wurde mit einem $\gamma$-Al$_2$O$_3$-Katalysator gearbeitet, der 0,018 Gew.-% SiO$_2$ enthielt. Aus dem Reaktorausgangsprodukt wurde reiner geruchsfreier Dimethylether praktisch quantitativ erhalten.

In Beispiel 2 wurde mit einem $\gamma$-Al$_2$O$_3$-Katalysator gearbeitet, der 0,005 Gew.% SiO$_2$ enthielt, jedoch mit einem Rückflußverhältnis von 1 : 0,4 und einem Einsatzprodukt von 80 Gew.% Dimethylether.
Es wurde wie in Beispiel 1 ein geruchsfreier Dimethylether praktisch quantitativ erhalten.

In Beispiel 3 wurde mit einem $\gamma$-Al$_2$O$_3$-Katalysator gearbeitet, der 0,4 Gew.% SiO$_2$ enthielt.
Es wurde wie in Beispiel 1 und 2 ein geruchsfreier Dimethylether praktisch quantitativ erhalten.

In Beispiel 4 wurde ein $\gamma$-Al$_2$O$_3$-Katalysator eingesetzt, der 0,025 Gew.% SiO$_2$ und 0,02 Gew.% Fe$_2$O$_3$ enthielt.
Es wurde unter den angegebenen Destillationsbedingungen ein geruchsfreier Dimethylether praktisch quantitativ erhalten.

In Beispiel 5 wurde wie in Beispiel 4 gearbeitet, jedoch war die Dimethylether-Kolonne mit Drahtgewebe gefüllt. Einsatzprodukt wurde an der erfindungsgemäß berechneten Stelle zugesetzt und Dimethylether und Verunreinigungen an den berechneten Stellen abgezogen.
Es konnte ein reiner, geruchsfreier Dimethylether erhalten werden.

In Vergleichsbeispiel 6 wurde mit einem Al$_2$O$_3$-Katalysator mit 0,020 Gew.% SiO$_2$ gearbeitet. Die Verunreinigungen wurdem am Kopf der 2. Kolonne abgezogen. Es konnte ein nahezu geruchsfreier Dimethylether gewonnen werden.

In Vergleichsbeipiel 7 wurde wie in Beispiel 6 gearbeitet, jedoch mit einem Katalysator der 6 Gew.% SiO$_2$ enthielt. Es konnte kein geruchsfreier Dimethylether gewonnen werden.

In Beispiel 8 wurde der reine Dimethylether in der ersten Kolonne bei 7 bar am 3. Boden (vom Kopf der Kolonne) bei einem Rückflußverhältnis von 1 : 3 gewonnen.
Das Einsatzprodukt wurde am 30. (gasförmig) und 36. (flüssig) Boden zugeführt (vom Kopf der Kolonne).
Die Verunreinigungen wurden am 18. Boden (vom Kopf der Kolonne) abgezogen.

Als Katalysator wurde ein $\gamma$-Al$_2$O$_3$-Katalysator eingesetzt, der 6 Gew.% SiO$_2$ enthielt.

Es wurde ein geruchsfreier, reiner Dimethylether erhalten.

In Beispiel 9 wurde gemäß Beispiel 8 gearbeitet, jedoch bei einem Rückflußverhältnis von 1 : 4.
Als Katalysator wurde ein Aluminiumsilikat-Katalysator verwendet.

Es wurde ein geruchsfreier, reiner Dimethylether erhalten.

In den folgenden Beispielen 10 - 14 wurde ein Einsatzprodukt in die Destillationskolonne zur Gewinnung reinen Dimethylethers eingesetzt und kontinuierlich destilliert, das in Gegenwart eines $\gamma$-$Al_2O_3$-Katalysators der 0,02 Gew.% $SiO_2$ enthielt, hergestellt wurde, bei einer Temperatur von ca. 260 - 280°C und einem Druck der um ca. 1-2 bar oberhalb des Dimethylether-Kolonnendrucks lag.

### Beispiel 10

Einer Kolonne mit 65 Ventilböden werden am 49. Boden (vom Kopf der Kolonne) 4000 kg/h eines Gemisches zugeführt, das aus 2400 kg Dimethylether 580 kg Methanol, 910 kg Wasser und 110 kg Verunreinigungen besteht.

Die Kolonne wird bei 8 bar betrieben. Das Rückflußverhältnis liegt bei 1 : 1,9.

Am Kopf der Kolonne werden ca. 30 m³ Abgas abgezogen, im wesentlichen bestehend aus $CO_2$, $N_2$, Kohlenwasserstoffen und kleinen Anteilen an Dimethylether.

Am 6. Boden der Kolonne (vom Kolonnenkopf) werden 2385 kg/h reiner Dimethylether abgezogen mit einem Methanolgehalt von 10 ppm.

Am 35. Boden (vom Kolonnenkopf) werden 90 kg/h an Verunreinigungen abgezogen. 10 kg/h an höher siedenden Komponenten (als Methanol), 580 kg/h Methanol und 910 kg/h $H_2O$ werden am Sumpf abgezogen und einer Kolonne zugeführt, in der das Methanol abdestilliert wird.

### Beispiel II

Beispiel lo wurde wiederholt, jedoch wurde die Fraktion, welche zwischen Dimethylether und Methanol siedet über einen Seitenstripper gefahren. Es wurden 2395 kg/h reiner Dimethylether erhalten und 8o kg/h Vorlauf am Stripperausgang.

### Beispiel 12

Einer Destillationskolonne mit 100 Ventilböden werden pro Stunde 61000 kg/h eines Gemisches zugeführt, das aus 55000 kg Methanol, 2000 kg Dimethylether, 3500 kg Wasser und 500 kg Verunreinigungen besteht. Das Rückflußverhältnis beträgt 1 : 7.

Die Zufuhr erfolgt am 35. Boden (vom Kolonnenkopf). Am 9. Boden (vom Kolonnenkopf) werden 1996 kg/h Dimethylether abgezogen.

Am 30. Boden (vom Kolonnenkopf) werden nach Strippen 302 kg/h Verunreinigungen abgezogen. Aus dem Sumpf der Kolonne werden 55000 kg/h Methanol, 3500 kg/h Wasser und 190 kg/h Verunreinigungen entnommen, die hauptsächlich aus höheren Alkoholen bestehen.

Der Kolonnendruck liegt zwischen 6 und 8 bar. Das über Kopf entnommene Abgas (12 kg) wird mit Methanol im Gegenstrom gewaschen.

### Beispiel 13

Einer Kolonne mit 70 Glockenböden werden am 48. Boden (vom Kolonnenkopf) 4000 kg/h eines Gemisches zugeführt, das aus 800 kg Dimethylether, 2825 kg Methanol, 300 kg Wasser und 75 kg zwischen Methanol und Dimethylether siedenden Verunreinigungen besteht. Ein Rückflußverhältnis liegt bei 1 : 2.

Am 4. Boden (vom Kolonnenkopf) werden 796 kg/h Dimethylether abgenommen. Am 40. Boden (vom Kolonnenkopf) werden 77 kg/h Verunreinigungen abgezogen.

Aus dem Sumpf werden 2825 kg/h Methanol und 300 kg/h Wasser entnommen.

### Beispiel 14

Einer Kolonne mit 45 Glockenböden werden am 34. Boden (vom Kolonnenkopf) 2000 kg/h eines Gemisches zugeführt, das 1750 kg Dimethylether, 100 kg Methanol und 100 kg Wasser enthält sowie 50 kg an Verunreinigungen. Die Kolonne wird bei einem Rückflußverhältnis von 1 : 0,5 betrieben.

Am 3. Boden (vom Kolonnenkopf) werden 1745 kg/h Dimethylether, am 29. Boden (vom Kolonnenkopf) 48 kg/h Verunreinigungen entnommen. Aus dem Sumpf werden 100 kg/h Wasser, 100 kg Methanol und 5 kg/h höher siedende Verunreinigungen abgezogen.

In den Beispielen 10 - 14 wurde ein hochreiner, geruchsfreier Dimethylether erhalten.

### Beispiel 15

Beispiel 13 wurde wiederholt, jedoch der Dimethylether am 18. Boden (vom Kopf der Kolonne) abgezogen. Verunreinigungen wurden am 45. Boden abgezogen.

Es wurde kein geruchsfreier Dimethylether erhalten.

### Patentansprüche

1. Verfahren zur kontinuierlichen Herstellung von reinem Dimethylether durch katalytische Dehydratisierung von Methanol bei einer Temperatur von l4o - 5oo°C und einem Druck von l bis 5o bar und destillative Aufarbeitung des Dehydratisierungsprodukts dadurch gekennzeichnet, daß das Dehydratisierungsprodukt in die Kolonne zur Gewinnung von reinem Dimethylether unterhalb des 25. Bodens (vom Kopf der Kolonne) an einem oder mehreren Böden in die Kolonne eingesetzt wird, daß der reine Dimethylether oberhalb des l5. Bodens, bevorzugt oberhalb des lo. Bodens (vom Kopf der Kolonne) an einem Boden oder mehreren Böden abgezogen wird, daß ein Verunreinigungen, die höher als Dimethylether und niedriger als Methanol sieden, enthaltendes Destillat an einem Boden oder mehreren Böden abgezogen wird, der (die) um mindestens 5 Böden oberhalb des (obersten) Zulaufs des den Dimethylether enthaltenden Einsatzgemisches und um mindestens einen Boden unterhalb des l5. Bodens (vom Kopf der Kolonne) liegt (liegen), und daß das Dehydratisierungsprodukt mindestens l Boden oberhalb des Kolonnensumpfes zugeführt wird, wobei bei einem Einsatzprodukt, das l - 5 Gew.% Dimethylether enthält, ein Rückflußverhältnis von l : l bis l : 25 gefahren wird, bei einem Einsatzprodukt, das 2o - 8o Gew.% Dimethylether enthält, ein Rückflußverhältnis von l:o,4 bis l : 5 gefahren wird, bevorzugt von l : l bis l : 2,5, bei einem Einsatzprodukt, das 6 - l9 Gew.% Dimethylether enthält, ein Rückflußverhältnis von l : o,4 bis l : 25 und bei einem Einsatzprodukt, das >8o Gew.% und <99 Gew.% Dimethylether enthält, ein Rückflußverhältnis von l: o,ol bis l : 5 gefahren wird.

2. Verfahren nach Anspruch l, dadurch gekennzeichnet, daß mit einem Katalysator vom $\gamma$-Al$_2$O$_3$ -Typ, der <l Gew.% SiO$_2$ enthält, dehydratisiert wird.

3. Verfahren nach den Ansprüchen l und 2, dadurch gekennzeichnet, daß mit einem Katalysator vom $\gamma$-Al$_2$O$_3$-Typ,der o,ool bis o,5 Gew.% SiO$_2$ enthält, dehydratisiert wird.

4. Verfahren nach den Ansprüchen l-3, dadurch gekennzeichnet, daß mit einem Katalysator vom $\gamma$-Al$_2$O$_3$-Type, der o,ool bis o,2 Gew.% SiO$_2$ enthält, dehydratisiert wird.

5. Verfahren nach den Ansprüchen l bis 4, dadurch gekennzeichnet, daß bei einer Temperatur von l4o$^\circ$ bis 45o$^\circ$ C und bei einem Druck von l bar bis 25 bar dehydratisiert wird.

6. Verfahren nach den Ansprüchen l-5, dadurch gekennzeichnet, daß bei einer Kontaktbelastung (LHSV) von o,2 - l6 l/l h, gearbeitet wird.

7. Verfahren nach den Ansprüchen l-6, dadurch gekennzeichnet, daß bei einer Kontaktbelastung (LHSV) von o,5 - l3,5 l/l h, gearbeitet wird.

8. Verfahren nach den Ansprüchen l-7, dadurch gekennzeichnet, daß die am Kopf der Destillationskolonne zur Gewinnung reinen Dimethylethers abgezogenen leichten Komponenten, die im allgemeinen CO$_2$, N$_2$, leichte Kohlenwasserstoffe und kleine Mengen an Dimethylether enthalten, in Gegen- oder Gleichstrom gewaschen werden, vorzugsweise mit Methanol.

9. Verfahren nach den Ansprüchen l-8, dadurch gekennzeichnet, daß die unterhalb des Abzugs des reinen Dimethylethers abgezogenen Verunreinigungen in einem Seitenstripper gestrippt werden und daß der so abgetrennte Dimethylether in die Destillationskolonne rückgeführt wird.

10. Verfahren nach den Ansprüchen l-9, dadurch gekennzeichnet, daß das Dehydratisierungsprodukt unterhalb des 25. Bodens an einem oder mehreren Böden je nach Bodenzahl zwischen dem 5o. und 25. Boden in die Kolonne eingesetzt wird.

11. Verfahren nach den Ansprüchen l-lo, dadurch gekennzeichnet, daß das Dehydratisierungsprodukt mindestens 5 Böden oberhalb des Kolonnensumpfes zugeführt wird.

12. Verfahren nach den Ansprüchen l - lo, dadurch gekennzeichnet, daß das Dehydratisierungsprodukt mindestens lo Böden oberhalb des Kolonnensumpfes zugeführt wird.

**Claims**

1. A process for the continuous production of pure dimethyl ether by catalytic dehydration of methanol at temperatures of between 140 and 500 $^\circ$C and pressures of between 1 and 50 bar and by purifying distillation of the dehydration product, which comprises:
   feeding the dehydration product to one plate or several plates below the 25$^{th}$ plate (counted from the top) of a column for producing pure dimethyl ether,
   withdrawing the pure dimethyl ether from one plate or several plates above the 15$^{th}$ plate (counted from the top) of the column, preferably above the 10$^{th}$,
   withdrawing the distillate containing impurities with higher boiling points than dimethyl ether and lower boiling points than methanol from one plate or several plates located at least five plates above the highest feed point for the dimethyl ether containing feedstock and at least one plate below the 15$^{th}$ plate (counted from the top of the column),

feeding the dehydration product at least one plate above the bottom of the column, wherein the reflux ratio is 1:1 to 1:25 if the feedstock contains 1-5 weight percent dimethyl ether, the reflux ratio is 1:0.4 to 1:5, preferably 1:1 to 1:25 if the feedstock contains 20-80 weight percent dimethyl ether, the reflux ratio is 1:04 to 1:25 if the feedstock contains 6-19 weight percent dimethyl ether, the reflux ratio is 1:0.01 to 1:5 if the feedstock contains more than 80 weight percent and less than 99 weight percent dimethyl ether.

2. A process in accordance with claim 1 wherein a gamma-$Al_2O_3$ catalyst containing less than 1 weight percent $SiO_2$ is used for the dehydration.

3. A process as claimed in claims 1 and 2 wherein a gamma-$Al_2O_3$ catalyst containing 0.0001 to 0.5 weight percent $SiO_2$ is used for the dehydration.

4. A process as claimed in any of the claims 1 to 3 wherein a gamma-$Al_2O_3$ catalyst containing 0.001 to 0.2 weight percent $SiO_2$ is used for the dehydration.

5. A process as claimed in any of the claims 1 to 4 wherein the dehydration is carried out at a temperature in the range of between 140 °C and 450 °C and at a pressure in the range of between 1 and 25 bar.

6. A process as claimed in any of the claims 1 to 5 wherein the LHSV is from 0.2 to 16 litres/litre and hour.

7. A process as claimed in any of the claims 1 to 6 wherein the LHSV is from 0.5 to 13.5 litres/litre and hour.

8. A process as claimed in any of the claims 1 to 7 wherein the light components removed at the head of the distillation column for producing pure dimethyl ether, said components generally containing $CO_2$, $N_2$, light hydrocarbons as well as a small amount of dimethyl ether, are washed in co-current or counter-current operation, preferably with methanol.

9. A process as claimed in any of the claims 1 to 8 wherein the contaminants removed below the withdrawal plate for pure dimethyl ether are stripped off in a lateral column and the dimethyl ether thus removed is recycled to the distillation column.

10. A process as claimed in any of the claims 1 to 9 wherein the dehydration product is fed below the 25th plate of the column to one or several plates between the 25th plate and the 50th, depending on the number of plates available.

11. A process as claimed in any of the claims 1 to 10 wherein the dehydration product is charged to the column at a point which is at least five plates above its bottom.

12. A process as claimed in any of the claims 1 to 10 wherein the dehydration product is charged to the column at a point which is at least ten plates above its bottom.

## Revendications

1. Procédé de production continue d'éther diméthylique pur par déshydratation catalytique de méthanol à une température de 140 à 500 °C et à une pression de 1 à 50 bars et traitement final par distillation du produit de déshydratation, caractérisé en ce que le produit de déshydratation est introduit au-dessous du 25ème plateau (compté depuis de la tête de la colonne) au niveau d'un ou plusieurs plateaux dans la colonne d'obtention de l'éther diméthylique pur, en ce que l'éther diméthylique pur est soutiré au-dessus du 15ème plateau, de préférence au-dessus du 10ème plateau (compté depuis la tête de la colonne) au niveau d'un ou plusieurs plateaux, en ce qu'un distillat contenant des impuretés qui bouillent plus haut que l'éther de diméthyle et plus bas que le méthanol est soutiré au niveau d'un ou plusieurs plateaux qui se trouvent au moins cinq plateaux au-dessus de l'admission (la plus haute) du mélange chargé contenant l'éther de diméthyle et au moins un plateau au-dessous du 15ème plateau (compté à partir de la tête de la colonne), et en ce que le produit de déshydratation est amené à au moins un plateau au-dessus du fond de la colonne, en sorte que lorsqu'on introduit un produit qui contient 1 à 5 % en poids d'éther de diméthyle, on pratique un rapport de reflux de 1:1 à 1:25, lorsqu'on introduit un produit qui contient 20 à 80 % en poids d'éther de diméthyle, on pratique un rapport de reflux de 1:0,4 à 1:5, de préférence de 1:1 à 1:2,5, lorsqu'on introduit un produit qui contient 6 à 19 % en poids d'éther de diméthyle, on pratique un rapport de reflux de 1:0,4 à 1:25 et lorsqu'on introduit un produit qui contient plus de 80 % en poids et moins de 99 % en poids d'éther de diméthyle, on pratique un rapport de reflux de 1:0,01 à 1:5.

2. Procédé suivant la revendication 1, caractérisé en ce qu'on effectue la déshydratation avec un catalyseur du type $\gamma$-alumine qui contient moins de 1 % en poids de $SiO_2$.

3. Procédé suivant les revendications 1 et 2, caractérisé en ce qu'on effectue la déshydratation avec un catalyseur du type $\gamma$-alumine qui contient 0,0001 à 0,5 % en poids de $SiO_2$.

4. Procédé suivant les revendications 1 à 3, caractérisé en ce qu'on effectue la déshydratation avec un catalyseur du type $\gamma$-alumine qui contient 0,001 à 0,2 % en poids de $SiO_2$.

5. Procédé suivant les revendications 1 à 4, caractérisé en ce qu'on effectue la déshydratation à une température de 140 à à 450°C et à une pression de 1 bar à 25 bars.

6. Procédé suivant les revendications 1 à 5, caractérisé en ce qu'on opère à une capacité du catalyseur (VSHL) de 0,2 à 16 1/1 h.

7. Procédé suivant les revendications 1 à 6, caractérisé en ce qu'on opère à une capacité du catalyseur (VSHL) de 0,5 à 13,5 1/1 h.

8. Procédé suivant les revendications 1 à 7, caractérisé en ce que les composants légers déchargés à la tête de la colonne de distillation pour l'obtention d'éther diméthylique pur, qui contiennent en général du $CO_2$, de l'azote moléculaire, des hydrocarbures légers et de petites quantités d'éther de diméthyle, sont lavés à contre-courant ou en courants parallèles, de préférence avec du méthanol.

9. Procédé suivant les revendications 1 à 8, caractérisé en ce que les impuretés déchargées au-dessous du point de soutirage de l'éther diméthylique pur sont éliminées par entraînement dans un épurateur latéral et l'éther diméthylique ainsi séparé est recyclé dans la colonne de distillation.

10. Procédé suivant les revendications 1 à 9, caractérisé en ce que le produit de déshydratation est introduit dans la colonne au-dessous du 25ème plateau au niveau d'un ou plusieurs plateaux entre le 50ème et le 25ème, selon le nombre de plateaux.

11. Procédé suivant les revendications 1 à 10, caractérisé en ce que le produit de déshydratation est amené au moins 5 plateaux au-dessus du fond de la colonne.

12. Procédé suivant les revendications 1 à 10, caractérisé en ce que le produit de déshydratation est amené au moins 10 plateaux au-dessus du fond de la colonne.

Figur 1

EP 0 270 852 B1

Figur 2

Figur 3

EP 0 270 852 B1